(19) **Europäisches Patentamt European Patent Office Office européen des brevets**

(11) **EP 3 658 689 B1**

(12) **EUROPEAN PATENT SPECIFICATION**

(45) Date of publication and mention of the grant of the patent:
**24.03.2021 Bulletin 2021/12**

(21) Application number: **17748700.6**

(22) Date of filing: **26.07.2017**

(51) Int Cl.:
*C12Q 1/6869* (2018.01)     *C12Q 1/6883* (2018.01)
*G16B 30/00* (2019.01)

(86) International application number:
**PCT/EP2017/068881**

(87) International publication number:
**WO 2019/020180 (31.01.2019 Gazette 2019/05)**

(54) **A METHOD FOR NON-INVASIVE PRENATAL DETECTION OF FETAL CHROMOSOME ANEUPLOIDY FROM MATERNAL BLOOD BASED ON BAYESIAN NETWORK**

VERFAHREN ZUM NICHT-INVASIVEN PRÄNATALEN NACHWEIS VON FETALER CHROMOSOMENANEUPLOIDIE AUS MÜTTERLICHEM BLUT AUF DER BASIS EINES BAYESISCHEN NETZWERKS

PROCÉDÉ DE DÉTECTION PRÉNATAL NON EFFRACTIF D'ANEUPLOÏDIE CHROMOSOMIQUE F TALE À PARTIR DU SANG MATERNEL SUR LA BASE D'UN RÉSEAU BAYÉSIEN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MD**

(43) Date of publication of application:
**03.06.2020 Bulletin 2020/23**

(73) Proprietor: **Trisomytest, s.r.o.**
**841 04 Bratislava - Karlova Ves (SK)**

(72) Inventors:
• **BUDIS, Jaroslav**
**85102 Bratislava (SK)**
• **DURIS, Frantisek**
**01001 Zilina (SK)**
• **KUCHARIK, Marcel**
**91501 Nove Mesto nad Vahom (SK)**

• **GAZDARICA, Juraj**
**83106 Bratislava (SK)**
• **SZEMES, Tomas**
**841 03 Bratislava (SK)**

(74) Representative: **Hak, Roman**
**Hak, Janecek & Svestka**
**Patent and Trademark Attorneys**
**U Pruhonu 5**
**170 00 Praha 7 (CZ)**

(56) References cited:
**WO-A1-2012/108920**    **WO-A1-2015/026967**
**WO-A1-2016/057901**    **WO-A1-2016/094853**
**US-A1- 2016 034 640**    **US-A1- 2016 371 428**

• **SUNG K. KIM ET AL: "Determination of fetal DNA fraction from the plasma of pregnant women using sequence read counts", PRENATAL DIAGNOSIS, vol. 35, no. 8, 3 August 2015 (2015-08-03) , pages 810-815, XP055215002, ISSN: 0197-3851, DOI: 10.1002/pd.4615**

**Description**

FIELD OF THE INVENTION

The invention relates generally to the field of non-invasive prenatal screening and diagnostics. The invention provides a method for detection of the presence or absence of aneuploidies, particularly trisomy and more particularly trisomy of chromosome 21, 18 and 13, sex and proportion of DNA fragments of fetus from the blood sample taken from mother in early stage of pregnancy.

BACKGROUND OF THE INVENTION

Nowadays, prenatal testing is an integral component of obstetric practice. The primary aim of prenatal testing is screening for fetal aneuploidies, such as trisomy of chromosome 21 (T21, Down syndrome), trisomy 18 (T18, Edwards syndrome), and trisomy 13 (T13, Patau syndrome). Although the majority of fetuses with aneuploidy result in termination during the development of the fetus, T21 has the highest survival rate, therefore, the prenatal detection of T21 is considered the most important prenatal genetic test for the purpose of prenatal screening or diagnostics. Reliable invasive prenatal tests are available, however, because of their risky nature, they are currently preformed only in high-risk pregnancies. Developing a reliable method for non-invasive prenatal testing (NIPT) for fetal trisomies seems to be recent challenge of the utmost importance in prenatal care.

The discovery of circulating cell-free fetal DNA (cffDNA) in maternal blood (Lo et al., Lancet 350:485-487, 1997) has offered the possibility for developing non-invasive processes that use fetal nucleic acids from a maternal peripheral blood sample to determine fetal chromosomal abnormalities. CffDNA constitutes approximately less than 10% of the total circulating cell-free DNA (cfDNA) in maternal plasma, however it has recently been found that the entire fetal genome, in the form of cffDNA, is present in maternal blood and thus it is very promising material for NIPT.

Several documents (Chiu et al 2008, Fan et al. 2008, Chiu et al 2011, Sehnert et al 2011, Lau et al 2102 and Bianchi et al. 2012, EP2183693, EP2366031, US8296076) disclosed the methods in which sequencing of DNA in maternal blood is used to obtain information on aberrant chromosome dosage in fetus. All methods mentioned above use analysis of the total cfDNA in maternal plasma without the need to isolate fetal-specific DNA, cffDNA. These methods are based on the detection of the extra copy of chromosome to distinguish normal cases from trisomy cases. In case of a fetus with trisomy, the number of copies of trisomic chromosome in the maternal blood is slightly higher in comparison with other autosomes. The advent of mass parallel DNA sequencing (MPS) permitted sequencing of extremely large quantities of DNA molecules and thus next-generation DNA sequencing (NGS) ave recently been used to detect non-invasive fetal trisomy from maternal blood.

Generally, the detection of fetal trisomy using NGS is done through the following process. First, a short region at one end of each DNA molecule of maternal plasma is sequenced and mapped against the reference human genome to determine the chromosomal origin of each sequence. Next, the amount of the sequenced tags from the chromosome of interest (e.g. chromosome 21) is compared with some kind of reference chromosome (usually another individual chromosome or selected group if chromosomes) and such value is compared between the examined sample and euploid control sample.

The crucial step of an analysis is to estimate proportion of DNA fragments originated from fetus, named fetal fraction. In case of low proportion diagnosed sample may be incorrectly classified as healthy based on abundance of maternal fragments.

EP2183693 (Lo, Y.-M.,D. et al., assigned to Chinese University of Hong Kong, HK) disclosed a method in which respective amounts of a clinically relevant chromosome and of background chromosome are determined from results of MPS and calculated percentage representation of sequences mapped to the chromosome 21. It was found that such a percentage representation is significantly higher in a sample of pregnant woman carrying a trisomy 21 fetus comparing to a woman with normal fetus. No z-score was determined as a decisive value in this method.

US8296076 (Fan, Ch. H.-M. and Quake, S.R., assigned to The Board of Trustees of the Leland Stanford Junior University, US) disclosed a method of fetal aneuploidy diagnostics based on sequencing. By counting the number of sequence tags mapped to a predefined window in each chromosome, the over- or under-representation of any chromosome in maternal plasma DNA contributed by an aneuploid fetus can be detected.

The methods of Chiu et al 2008, Chiu et al 2011, Sehnert et al 2011, Lau et al 2012 are based on whole genome sequencing using MPS and z-score determination. Although z-score is widely accepted as the standard parameter used for detection of aneuploid samples, there are differences in its calculation. Chiu et al. 2011 disclosed an approach using reads mapped to all chromosomes used as reference (with exception of the chromosome 13, 18 and 21) for z-score calculation, Lau et al. 2012 disclosed an approach using reads mapped to some specific chromosome, e.g. 14 as reference for T21 trisomy, and Sehnert et al. 2011 chose chromosome 9 to be the optimal internal reference for the chromosome 21.

Generally, there are three main stages of the determination of aneuploidy of fetus from a maternal blood, plasma or serum sample: 1) preparation of DNA sample and DNA library, 2) sequencing, and 3) analysis of the sequence data. Sequencing made remarkable progress in the past few years, however, in the first and the third stage there is a space for improving that might have low cost and big impact on the quality of the testing.

There are various approaches how to improve the first stage, as disclosed for example in EP2366031 (Rava, R.P. et al., assigned to Verinata Health, Inc., US). The document disclosed a method for prenatal screening and diagnostics of fetal chromosomal aneuploidy on the basis of NGS comprising a novel protocol for preparing sequencing libraries from a maternal sample. The novel approach in preparing sequencing libraries comprises the consecutive steps of end-repairing, dA-tailing and adaptor ligating said nucleic acids, and wherein said consecutive steps exclude purifying the end-repaired products prior to the dA-tailing step and exclude purifying the dA-tailing products prior to the adaptor-ligating step. The method allows for determining copy number variations (CNV) of any sequence of interest. No z-score was determined as a decisive value in this method.

Another improvement was disclosed in WO 2011/051283 (Benz, M. et al., assigned to Lifecodexx, AG, DE). The method for non-invasive diagnosis of chromosomal aneuploidy disclosed therein is improved by the enrichment and quantification of selected cfDNA sequences in a maternal blood sample.

Since male fetus have different pair of sex chromosomes (X and Y) than mother (two copies of the X), fetal fraction is routinely estimated from abundance of fragments in these chromosomes. This approach is however limited to samples with a male fetus. Fraction of female fetus may be estimated from characteristics differing between fetal and maternal fragments.

Differences in fragment localization across genome were utilized in the SeqFF method (Kim et al. 2015). They demonstrate that fetal fractions vary in different regions of genome and significantly correlate with GC content and presence of exonic regions. The method therefore uses normalized, binned counts as predictor variables and estimates fetal fraction using standard multivariate regression models.

SANEFALCON method (Straver et al. 2016) uses more detailed information of fragment origin based on different mechanisms of fragment degradation. They used proportion of consistent fragments with precalculated genomic locations of nucleosomes as fetal fraction predictor, based on assumption that maternal fragments originate more often on positions of nucleosomes than fetal fragments.

Another promising characteristic is a length of a fragment. Since DNA fragments of fetal origin tend to be shorter, profiles of maternal and fetal fragment lengths differ significantly and may be used as predictor of fetal fraction. The length-based method has been proposed in Yu et al. 2014 WO-A-2016/094853 discloses a method of using cell-free DNA fragment size to determine copy number variations (CNVs).

Fetus genome inherited genomic information evenly from father and mother. Based on origin, it differs in certain positions, most notably in point changes of nucleotides called SNP. Fragments that are consistent with SNPs specific to father are most likely of fetal fraction and may be used in the prediction. The method FetalQuant (Jiang et al. 2016) uses this information, however requires another laboratory assay for genetic map of parents. Application of the method is thus too time-consuming and expensive for routine diagnosis. In spite of existence of several methods for non-invasive detection of fetal aneuploidy there is still a need for alternative method that would be at least as sensitive and specific as the present methods and less costly at the same time. In addition, current methods for fetal fraction prediction are still limited by their precision or requirement for additional laboratory assay.

The present invention provides alternative and reliable method that is applicable to the practice of non-invasive prenatal screening for aneuploidies such as trisomy of chromosome 13, 18 or 21. It provides simultaneous detection of aneuploidy, sex and fetal fraction using united model which may be easily expanded to another chromosomal aneuploidies and disorders, such as copy number variations. The model utilizes various sources of information of DNA fragment origin, such as chromosome location and length of DNA fragment, and may be gradually extended to incorporate other sources to reach even better accuracy.

SUMMARY OF THE INVENTION

The following description will explain the main features of the method of the present invention, which is defined by the appended claims. The skilled person will get full understanding of the present invention from the following description together with the examples, wherein some specific features and aspects will be explained in more details.

The technical and scientific terms used herein have the same meaning as commonly understood by the persons skilled in the art of medicine, molecular genetics, prenatal non-invasive diagnostics, molecular biology and bioinformatics. Some specific terms are explained bellow.

*Aneuploidy* is used in the common sense known to the person skilled in the art, it means an imbalance of genetic material caused by a loss or gain of a whole chromosome or part of chromosome. In other words, it means the presence of the entire excessive chromosome or the absence of full chromosome or partial chromosome duplication or deletions of a significant size (> 1 kbp). Specifically, *T21, T18* and *T13* denote the most common types of autosomal trisomies that survive to birth in humans, which is trisomy of chromosome 21 resulting in Down's syndrome, trisomy of chromosome 18 resulting in Edwards syndrome and trisomy of chromosome 13 resulting in Patau syndrome.

The term *massively parallel sequencing* (MPS), used in combination with *new generation sequencing* (NGS) means techniques, that are well known to the persons skilled in the art, of sequencing of huge amount, i.e. in millions or ten of millions order, fragments of DNA, as it is practiced in the art using Illumina or IonTorrent analyzers. Protocols for whole genome sequencing are known to the skilled person.

Sequence *reads* being the short DNA sequences obtained from NGS sequencing, however, long enough (*e.g.* at least about 30 bp) to serve as sequence *tags*, i.e. that can be assigned unambiguously to one of the chromosomes (1-22, X, Y). Small degree of mismatch can be usually allowed (1bp). In fact, the tags are assigned or rather mapped reads. The tags are uniquely mapped to a reference genome *i.e.* they are assigned to a single location to the reference genome. Tags that can be mapped to more than one location on a reference genome *i.e.* tags that do not map uniquely, are excluded from the analysis.

*Mapping* means an alignment of the sequence information from NGS (i.e. DNA fragment the genomic position of which is unknown) with a matching sequence in reference human genome. This can be done be several ways, we used the method of Liu et al 2014. As used herein, the term *human reference genome* or *reference genome* refers to hg19 sequence.

As used herein, the terms *aligned* or *alignment* refer to one or more sequences that are identified as a match in terms of the order of their nucleic acid molecules to a known sequence from a reference genome. Such alignment can be done manually or by a computer algorithms that are well known to the persons skilled in the art of molecular biology and bioinformatics. The matching of a sequence read in aligning can be a 100% sequence match or less than 100% (non-perfect match).

*Fetal fraction* is the proportion of DNA fragments originated from the fetus compared to all sequenced fragments.

*GC content bias* describes the dependence between fragment count (read coverage) and GC content found in Illumina sequencing data. This bias can dominate the signal of interest for analyses that focus on measuring fragment abundance within a genome, such as copy number estimation. The bias is not consistent between samples; and there is no consensus as to the best methods to remove it in a single sample. There are many proposed approaches to correcting this bias, such as Benjamini and Speed 2012 or Liao et al. 2014.

*Bayesian network* is probabilistic model that represents a set of random variables and their conditional dependencies via a directed acyclic graph.

*Posterior probability* of a random event means the conditional probability that is assigned after the relevant evidence or background is taken into account. Similarly, the *posterior probability distribution* is the probability distribution of an unknown quantity, treated as a random variable, conditional on the evidence obtained from an experiment or survey. "Posterior", in this context, means after taking into account the relevant evidence related to the particular case being examined.

*Expectation-maximization (EM) algorithm* is an iterative method allowing finding of maximum likelihood or maximum a posteriori (MAP) estimates of parameters in statistical models, where the model depends on unobserved latent variables.

The present invention, as defined in the claims, relates to the method for determining aneuploidy and sex of fetus, from a maternal blood, plasma or serum sample comprising a mixture of fetal and maternal nucleic acid molecules, wherein the mixture of fetal and maternal nucleic acid molecules are cell-free DNA molecules, said method comprising four main stages: 1) obtaining and treating samples of maternal blood, 2) preparation of DNA sample and DNA library, 3) sequencing, and 4) analysis of the sequence data to obtain a prediction. The important part of the method is preparation of the training data, i.e. analysis of the set of euploid samples, that are processed in the same way as test samples. The minimum training set of samples should comprise mother with healthy, i.e. euploid male fetus and mother with healthy, i.e. euploid female fetus, preferably in the number of about one hundred of each. The starting point of the present method is analysis of the maternal sample, i.e. peripheral blood, thus the method is non-invasive. The peripheral blood comprises circulating cell free DNA (cfDNA) that is a mixture of fragments of maternal DNA and fetal DNA (cffDNA), further named also DNA fragments or briefly fragments. CffDNA is what matters, therefore fetal DNA can be enriched (by selection of the shorter fragment, either *in silico* or physical selection). Then total DNA sample (still comprising both maternal and fetal DNA, i.e. it is a mixed sample) is subjected to the massively parallel sequencing by NGS approach, to obtain huge number of short sequence reads. These reads serve as sequence tags, i.e. they are mapped to a certain genomic region or chromosome.

The method of the invention assigns posterior probability of fetal origin to each DNA fragment, using implemented fragment length and chromosomal position as selected features of DNA fragments, however it is straightforward to iteratively extend model with other types of features (for example fragment's distance from the nearest coding region, its GC content and/or single nucleotide polymorphism information), making it more precise. The fragments with high probability of being of fetal origin have larger effect on the test result than fragments with low such probability

The main improvement and advantage of the present method lies in the step of the analysis and processing of the sequence data. Prediction of sex, euploidy/aneuploidy and fetal fraction are highly interdependent, however described state-of-art methods process them independently, which may lead to their inconsistencies. In contrast, the method according to the present invention processes the properties of observed cfDNA fragments simultaneously which results in more reliable, unified prediction.

The Bayesian network (named also Bayesian model), which is a base of the present method, allows natural designing of detection method of other genomic abnormalities, such as sex chromosome aberrations, microaberrations and non-standard aneuploidies, based on position, range and type of abnormality defined by a person skilled in the art.

One aspect of the present invention relates to a method for determining aneuploidy and sex of fetus from a maternal blood, plasma or serum sample, together with fetal fraction, as explained in details bellow and as defined in the attached claims.

Nowadays, there is a practical requirement to automatize the methods of the prenatal tests or diagnostics. The method of the present invention can be largely automatized. At least the bioinformatics part of the method (i.e. processing of sequencing data and all subsequent determinations and calculations) may be performed using suitable computer system, such as PC equipped with a processor, peripheral input/output devices (e.g. ports, interfaces), memories (e.g. system memory, hard disk), keyboard, monitor, mouse etc., and a specific software, programme for instructing the computer system to perform specific step. Preferably, the computer system is in data communication with the sequencing system providing the sequence data, preferably in the form of plurality of sequence reads (by a wire or wireless networking, bluetooth, internet, cloud etc.). It means that the computer system is configured for receiving sequence data from the sequencing system. The suitable computer systems as well as means for connection with sequencing system are well known to the persons skilled in the art.

At least part of the method, specifically the bioinformatics part of the method, can be implemented as a software code, i.e. a plurality of instructions (computer program) to be executed by a processor of a computing system. The code may be comprised in the computer readable medium for storage or transmission such as for example RAM, ROM, harddrive, SDS, CD, DVD, flash memory etc. Furthermore, the code may be transmitted via any suitable wired, optical or wireless network, for example via internet. For example the whole computer program can be downloaded by the user (customer) via the internet.

Therefore, another aspect of the present invention relates to the computer implemented method comprising all steps of calculations and determinations needed for processing of the input data (sequencing data) into the output parameter(s) characterizing the diagnosis condition (sex, presence/absence of aneuploidy, fetal fraction).

Still another aspect of the present invention relates to a computer program product comprising a computer readable medium comprising a plurality of instructions for controlling a computing system to perform at least a portion of the method according to the invention, preferably portion thereof starting with the step of receiving sequence information from the random sequencing step performed with automated sequencing system. The computer program mentioned above can be advantageously introduced into computer of the sequencing system.

The subject matter of the present invention is defined in the claims as attached.

The features and advantages of the present invention will be understood by the person skilled in art from the following detailed description, examples and figures.

BRIEF DESCRIPTION OF THE DRAWINGS

Fig. 1. A general scheme of the method demonstrating the basic steps and the iterative nature of the processing data obtained from sequencing.

Fig. 2. A scheme of Bayesian network (Bayesian model).

Figure 3. Estimated fragment length distributions for maternal and fetal DNA fragments. The empirical distribution of sequenced sample with estimated fetal fraction 25.6% is demonstrated.

Figure 4. Comparison of fetal fraction estimated from abundance of reference chromosomes and the Bayes method according to the invention.

DETAILED DESCRIPTION OF THE INVENTION

1. THE METHOD

In this novel method, we look at the cfDNA fragments (cfDNA fragments or briefly fragments) sequenced from maternal blood (or plasma or serum) as a mixture of cfDNA fragments of fetal and maternal origin. We examine several features of these fragments that are differently distributed in mother and fetus, and we use these differences to determine the most probable composition of this mixture.

To determine this composition, we use the expectation-maximization (EM) algorithm. Bayesian network is a probabilistic graphical model, that represents a set of random variables that can be observed (fragment length and chromosomal position of the fragment), which allows to predict posterior probability of an unobserved variable (fetal origin of fragment). We designed a Bayesian network to describe the mixture of fetal and maternal cfDNA fragments in maternal plasma with several features each with different distribution of values depending on the fragment's origin (i.e. fetal or maternal). The distributions of chromosome of origin (= chromosomal position of the fragment or fragment location) and fragment length for fetal and maternal samples are initially trained from set of train samples and then adjusted by the EM algorithm. The output of the novel method is classification of the examined sample into one of the four categories, such as euploid male, aneuploid male, euploid female, aneuploid female, i.e. the most probable class for the case with the most probable fetal fraction of cfDNA fragments with respect to the NGS data. Additionally, the method can be extended to include twins or triplets into consideration as well.

We note that the method can include additional features of the fragments (such as fragment's distance from the nearest coding region, its GC content etc.) in an analogous manner, and the chromosomal aberrations under consideration include all autosomes as well as sex chromosomes.

More specifically, the method consists of the following steps. For each case, for example one specific trisomy detection (for example T21) and each possible category (aneuploid male, aneuploid female, euploid male, euploid female) do

1. Gather and process training samples
2. Train initial distributions regarding the fragment length and chromosome of origin
3. Construct a Bayesian network that includes the fragment features: fragment length (LEN) and chromosome of origin (CHROM)
4. Run EM algorithm on this Bayesian network on training samples
5. Based on the EM output

   a. classify fragments from the sample as maternal or fetal
   b. adjust chromosome of origin and fragment length distributions

6. Repeat steps 4 and 5 until parameters of trained distribution does not significantly change between iterations, e.g. sum of differences is below 0.000001
7. Calculate the probability of the selected features of DNA fragments in the final Bayesian network;

After calculation of posterior probabilities on all categories (aneuploid male, aneuploid female, euploid male, euploid female), we select the most probable case as a test result or diagnosis.

## 1.1 SELECTING AND PROCESSING TRAIN SAMPLES

The following training sets may be used in the method of the invention for the detection of T13, T18 and T21 trisomies. The samples from all sets are sequenced, and the resulting fastq files are mapped to human genome. Optionally, after mapping, GC correction is applied.

1. Select a training set of pregnant women with single euploid female fetus, denote it $T_{hx}$
2. Select a training set of pregnant women with single euploid male fetus, denote it $T_{hy}$
3. Select a training set of pregnant women with single T13, T18 and T21 trisomic female fetus, denote it $T_{t13x}$, $T_{t18x}$ and $T_{t21x}$, respectively
4. Select a training set of pregnant women with single T13, T18 and T21 trisomic male fetus, denote it $T_{t13y}$, $T_{t18y}$ and $T_{t21y}$, respectively
5. Define euploid training set as $T_h = T_{hx} \cup T_{hy}$
6. Select a training set of adult males, denote it with $T_m$
7. Select a training set of non-pregnant females, denote it with $T_f$
8. For each sample from each training set apply the following steps

   a. Raw sequences are mapped to the unmasked reference human genome (hg19) using a Bowtie2 algorithm (Langmead and Salzberg, 2012)
   b. Only sequence reads that could be mapped to just one genomic location with at most one mismatch are retained (Ehrich et al., 2011; Sehnert et al., 2011)

    c. Splitting the reference genome into 20kb subsequent and non-overlapping bins and distributing the mapped sequence reads to said bins according to their mapping location

    d. Optionally, number of reads is corrected for GC content bias according to Loess and PCA correction (Zhao 2015)

## 1.2 BUILDING FRAGMENT FEATURE DISTRIBUTIONS

### 1.2.1 Chromosomes

One can observe that there are differences between distributions of cfDNA fragments mapped to chromosomes for pregnant woman with male and female fetus (most notable on the X and Y chromosomes). Similarly, there are differences in such distributions for euploid and aneuploid fetus (e.g., on chromosome 21 for T21 trisomic fetus). These observations can be used to determine the most probable sources of the cfDNA mixture in maternal plasma, that is, whether the woman is carrying male or female fetus and whether it is healthy or aberrant (e.g. aneuploid, e.g. trisomic), by looking at the mixture distribution of cfDNA fragments.

    **Assumption 1.1.** *It is assumed that the distribution of cfDNA fragments from maternal plasma to autosomes is equal for both mother and euploid fetus. If, additionally, the fetus is girl, this equality extends to all chromosomes. If the fetus is boy, the distribution to X and Y chromosomes will be different than that of the mother.*

    **Assumption 1.2.** *It is assumed that the distribution of cfDNA fragments from maternal plasma of the euploid male fetus to chromosomes is equal to that of healthy male.*

**Steps:**

    1. Calculate fractional autosomal representation (FAR) for each sample in the set $T_h$ (see *Calculation of FAR* below)

    2. Calculate fractional chromosomal representation (FCR) for each sample in the sets $T_m$ and $T_{hx}$ (see *Calculation of FCR)*

    3. Calculate the mean FAR for each autosome (see *Calculation of mean FAR for autosomes*) in $T_h$

    4. Calculate the mean FCR for each chromosome (see *Calculation of mean FCR for chromosomes*) in $T_m$ and $T_{hx}$

    5. Build chromosome distributions CHR-DIST (see *Building chromosome distribution*)

**Calculation of FAR**

Let $M = (m_{i,j})_{k \times 22}$ be a matrix associated with the training set $T_h$, where $k$ is the size of $T_h$ and $m_{i,j}$ holds the number of fragments mapped (and GC corrected) to *the* $j^{th}$ autosome for $i^{th}$ sample of $T_h$. Let $M_i$ denote the sum of the $i^{th}$ row of the matrix $M$. The FAR values for each autosome of the $i^{th}$ sample (i.e., $i^{th}$ row if the matrix M) are defined as $\frac{m_{i,x}}{M_i}$, where $x$ denotes the number of autosome (i.e., $x \in \{1, 2, ...,22\}$).

We will denote a matrix of FAR values of the samples from the training set $T_h$ as $F_h$.

**Calculation of FCR**

Let $N = (n_{i,j})_{k \times 24}$ be a matrix associated with the training set $T_m$, where $k$ is the size of $T_m$ and $n_{i,j}$ holds the number of fragments mapped (and GC corrected) to the $j^{th}$ chromosome for $i^{th}$ sample of $T_m$ (we let the chromosome X have number 23 and chromosome Y have number 24). Let $N_i$ denote the sum of the $i^{th}$ row of the matrix $N$. The FCR values for each chromosome of the $i^{th}$ sample (i.e., $i^{th}$ row if the matrix N) are defined as $\frac{n_{i,x}}{N_i}$, where $x$ denotes the number of autosome (i.e., $x \in \{1, 2, ...,24\}$).

We will denote a matrix of FCR values of the samples from the training set $T_m$ as $F_m$. The FCR values of the samples from the training set $T_{hx}$ can be calculated analogously, and we will denoted them with $F_{hx}$.

**Calculation of mean FAR for autosomes**

Let $F_h = (f_{i,j})_{k \times 22}$ be a matrix associated with the FAR values of the training set $T_h$, where $k$ is the size of $T_h$ and $f_{i,j}$ holds the FAR value of *the $j^{th}$* autosome for $i^{th}$ sample of $T_h$. The mean FAR value of *the $j^{th}$* autosome is defined as mean value *of $j^{th}$* column of the matrix $F_h$.

We will denote the mean FAR values of the $T_h$ set with $FAR_j$, where $j \in \{1, 2, ...,22\}$ is the number of autosome.

**Calculation of mean FCR for chromosomes**

Let $F_m = (f_{i,j})_{k \times 24}$ be a matrix associated with the FCR values of the training set $T_m$, where $k$ is the size of $T_m$ and $f_{i,j}$ holds the FCR value of the $j^{th}$ chromosome for $i^{th}$ sample of $T_m$. The mean FCR value of *the $j^{th}$* chromosome is defined as mean value *of $j^{th}$* column of the matrix $F_m$. Analogously for the matrix $F_{hx}$.

We will denote the mean FCR values of the sets $T_m$ and $T_{hx}$ with $FCR_j^m$ and $FCR_j^{hx}$, respectively, where $j \in \{1, 2, ...,24\}$ is the number of chromosome.

1.2.2 Building chromosome distributions

In the present model a huge number of chromosome distributions can be defined based on the nature of the genetic disorder which we would like to observe. Preferably, we will focus on the aneuploidies of single whole autosomes (e.g., T13, T18, T21), but the model can encompass aneuploidies of multiple autosomes as well as parts of these autosomes. For the sake of completeness, we will describe the building of chromosome distribution in the most general case.

A chromosomal distribution consists of 24 numbers, one for each chromosome, describing the probability that some cfDNA fragment will be mapped to a particular chromosome.

Let $FAR = (FAR_1, FAR_2, ..., FAR_{22})$ be mean FAR values of the training set $T_h$. Let $FCR^m = (FCR_1^m, FCR_2^m, ..., FCR_{24}^m)$ and $FCR^{hx} = (FCR_1^{hx}, FCR_2^{hx}, ..., FCR_{24}^{hx})$ be mean FCR values of the training sets $T_m$ and $T_{hx}$, respectively.

The chromosomal distribution for both healthy woman (i.e., woman herself) and healthy female fetus is given by $FCR^{hx}$ (see *Assumption 1.1* above). In the following text we will use the symbols $CHR$ - $\text{DIST}_{x, h}$ and $CHR$ - $\text{DIST}_{f, h}$ for the chromosomal distribution of healthy female fetus and healthy female herself, respectively. The chromosomal distribution of the healthy male fetus is calculated as follows. First, we define a normalizing constant $NK$ as

$$NK = \sum_{j=1}^{22} FCR_j^m.$$

Let

$$t_{23} = \frac{FCR_{23}^m}{NK}$$

$$t_{24} = \frac{FCR_{24}^m}{NK}.$$

and

$d = (FAR_1, FAR_2, ..., FAR_{22}, t_{23}, t_{24})$.

With s denoting the sum of the vector d, the distribution of the healthy male fetus is given by

$$D = \left( \frac{FAR_1}{s}, \frac{FAR_2}{s}, ..., \frac{FAR_{22}}{s}, \frac{t_{23}}{s}, \frac{t_{24}}{s} \right).$$

In the following text we will use the symbol $CHR$ - $\mathrm{DIST}_{y,h}$ for the chromosomal distribution of healthy male fetus.

Building distribution for chromosomal aberrations (e.g. aneuploidies) is rather simple. First, we choose the target of the aberration, i.e., mother or fetus. Then, we choose the affected autosomes. Finally, we choose for each affected autosome the degree of aberration indicating how large proportion of the affected autosome is missing or multiplicated. Thus, let $t \in \{f, x, y\}$ be the target (we are already distinguishing male and female fetus). Furthermore, let

$$a = \mathbb{R}^{24},$$ $a = (a_1, a_2, ..., a_{24})$, be a vector indicating the extent of autosomal aberrations ($a_i = 0$ if the $i^{th}$ autosome is unaffected, $a_i = -2$ if the both copies of the $i^{th}$ autosome are missing, $a_i = 1$ if the $i^{th}$ autosome is trisomic, and $a_i = -1$ if the $i^{th}$ autosome is monosomic). With

$CHR\text{-}DIST_{t,h} = (ch_1, ch_2, ....,ch_{24})$

the aberrant chromosomal distribution is given by

$$CHR - DIST_{t,a} = \left( \frac{ch_1 \left(1 + \frac{a_1}{2}\right)}{s}, \frac{ch_2 \left(1 + \frac{a_2}{2}\right)}{s}, ..., \frac{ch_{24} \left(1 + \frac{a_{24}}{2}\right)}{s} \right),$$

where

$$s = ch_1 \left(1 + \frac{a_1}{2}\right) + ch_2 \left(1 + \frac{a_2}{2}\right) + ... + ch_{24} \left(1 + \frac{a_{24}}{2}\right).$$

1.2.3 Fragment lengths

> **Assumption 1.3.** *It was observed* (Stephanie et al., 2014) *that fetal cfDNA fragments are, on average, shorter than maternal cfDNA fragments, that is, the lengths of maternal fragments are distributed differently than that of fetus.*

> **Assumption 1.4.** *It is assumed that the distribution of lengths of cfDNA fragments mapped to autosomes for euploid male and female fetus is equal. These two distribution are not, however, equal when all chromosomes are considered. There is a subtle difference as the two X chromosomes of the female fetus produce more shorter reads than X and Y chromosomes of the male fetus. Thus, a sample from pregnant woman with female fetus is expected to have more shorter reads than from a pregnant woman with male fetus.*

> **Assumption 1.5.** *It is assumed that the distribution of lengths of cfDNA fragments mapped to autosomes for pregnant and non-pregnant women is equal (here we mean the pregnant women as such, not as a mixture of fetal and maternal cfDNA).*

> **Assumption 1.6.** *It is assumed that the sequenced fragments are not longer than 1000bp.*

**Steps:**

> 1. Calculate fractional length distribution (FLD) from samples in training set $T_f$ (see *Calculate FLD from $T_f$* below)
> 2. Calculate fractional length distribution of fragments mapped to Y chromosome from samples in training set $T_{hy}$ (see *Calculate FLD from $T_{hy}$*)
> 3. Build fragment length distribution for 0% fetal fraction $LEN$ - $DIST_0$ (see *Building LEN - DIST$_0$*)
> 4. Build fragment length distribution for 100% fetal fraction $LEN$ - $DIST_1$ (see *Building LEN - DIST$_1$*)

Note that in these distributions we do not distinguish between male or female fetus, as well as euploid or aberrant fetus. We note that trisomic female fetus will have, on average, more shorter reads than euploid male fetus. However, at this point we choose to neglect these differences.

**Calculate FLD from $T_f$**

Let $M = (m_{i,j})_{k \times 1000}$ be a matrix associated with the training set $T_f$, where $k$ is the size of $T_f$ and $m_{i,j}$ holds the number of mapped (and GC corrected) fragments of length j for $i^{th}$ sample of $T_f$. Let $M_i$ denote the sum of the $i^{th}$ row of the matrix $M$. Then, the fractional length distribution of the $i^{th}$ set is given by $\left( \dfrac{m_{i,1}}{M_i}, \dfrac{m_{i,2}}{M_i}, ...., \dfrac{m_{i,1000}}{M_i} \right)$.

We will denote a matrix of FLD values of the samples from the training set $T_f$ as $L_f$.

**Calculate FLD from $T_{hx}$**

This step is basically identical to *Calculate FLD from $T_f$* with the only difference that the training set is $T_{hy}$ and that we are interested only in fragments mapped to Y chromosome.

We will denote a matrix of FLD values of the samples from the training set $T_{hy}$ as $L_{hy}$.

**Building *LEN* - $DIST_0$**

Let $L_f = (l_{i,j})_{k \times 1000}$ be a matrix of FLD values of the training set $T_f$, where $k$ is the size of $T_f$. Let $L_j$, $j \in \{1, 2, ...,1000\}$, denote the mean value of the $j^{th}$ column of the matrix $L_f$. Then, the fragment length distribution for 0 % fetal fraction is given by

$$LEN - DIST_0 = (L_1, L_2, ..., L_{1000}).$$

**Building *LEN* - $DIST_1$**

Let $L_{hy} = (l_{i,j})_{k \times 1000}$ be a matrix of FLD values of the training set $T_f$, where $k$ is the size of $T_{hy}$. Let $K_j$, $j \in \{1, 2, ...,1000\}$, denote the mean value of the $j^{th}$ column of the matrix $L_{hy}$. Then, the fragment length distribution for 100 % fetal fraction is given by

$$LEN - DIST_1 = (K_1, K_2, ..., K_{1000}).$$

1.3 THE BAYESIAN NETWORK AND EM ALGORITHM

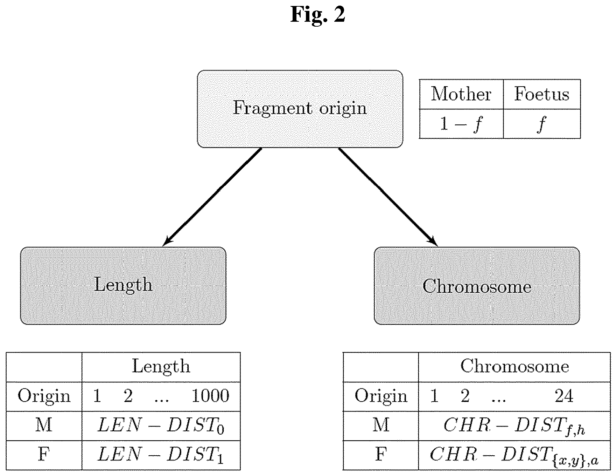

In the network of fetal/maternal mixture of cfDNA fragments we examine the fragment's length and chromosome of origin (The Bayesian network used to this purpose is shown on Fig. 2).

Note that we have, in fact, four Bayesian networks that differ in the values of CHROM and LEN distributions for classes defined as aneuploid male, aneuploid female, euploid male and euploid female.

By *CHR - $DIST_{\{x,y\},a}$* we mean that we use either *CHR - $DIST_{x,a}$* for female fetus (with autosomal aberrations given by *a*), or we use *CHR - $DIST_{y,a}$* for male fetus (with autosomal aberrations given by *a*).

On this Bayesian network, we run the EM algorithm. The observed variables are fragment length and chromosome of origin for all sample's fragments. The fragment origin (mother, fetus) is one hidden or unobserved variable which is predicted by the EM inference. The parameters of the probability model are *f*, *LEN - $DIST_0$*, *LEN - $DIST_1$*, *CHR - $DIST_{f,h}$*, and *CHR - $DIST_{\{x,y\},a}$*.

Let **x** be all observed values in all sample's fragments, let Z denote the hidden variable for all fragments, and let $\theta$ be all the parameters for the probability model. Then, the EM algorithm can be summarized in this equation

$$\theta^{(i+1)} = \arg \max_{\theta} \sum_z P(Z = z | x, \theta^{(i)}) L(\mathbf{x}, Z = z | \theta^{(i)}), \qquad (1.1)$$

where P stands for probability and L for log likelihood. More particularly, let $\theta^{(0)}$ be the set of initial parameters determined as described in *Section 1.2*. Also, initially we will set $f^{(0)} = 0.5$. For the next iteration, we have

$$f^{(1)} = \sum_{j=1}^{N} Pr\left[\text{Fragment origin} = \text{fetal} \mid \text{chromosome} = ch_j, \text{ length} = l_j\right] / N,$$

where N is the total number of reads. These probabilities can be computed from the Bayesian network. Assuming that fragment length is independent of the chromosome, it follows that

$$f^{(1)} = \frac{1}{N} \sum_{j=1}^{N} \frac{Pr\left[\text{chrom} = ch_j \mid \text{fetal}\right] Pr\left[\text{len} = l_j \mid \text{fetal}\right] Pr\left[\text{fetal}\right]}{\sum_{ori \in \{fetal, maternal\}} Pr\left[\text{chrom} = ch_j \mid ori\right] Pr\left[\text{len} = l_j \mid ori\right] Pr\left[ori\right]}$$

The probabilities $Pr$ [chrom = $ch_j$ |fetal] and $Pr$ [len = $l_j$ | fetal] in the first iteration can be obtained from the pre-trained distributions LEN-DIST and CHR-DIST (values $CHR\text{-}DIST_{\{x,y\},a}[ch_j]$ and $LEN\text{-}DIST_1[l_j]$). Analogously, for values $P_r$[chrom = $ch_j$|maternal] and $Pr$[len = $l_j$|maternal]. Finally, $Pr[fetal]$ = $f^{(0)}$ and $Pr[maternal]$ = 1 -$f^{(0)}$ in this iteration.

Similarly, we can find new expected values for vectors $LEN\text{-}DIST_0$, $LEN\text{-}DIST_1$, $CHR\text{-}DIST_{f,h}$, and $CHR\text{-}DIST_{\{x,y\},a}$. For example,

$$LEN - DIST_0[1]^{(1)} = \sum_{j: len_j = 1} Pr\left[\text{Fragment origin} = \text{fetal} \mid \text{length} = 1, \text{ chromosome} = ch_j\right]$$

which value can be calculated analogously to the calculation of $f^{(1)}$.

Equations alternate in iterative manner. At first, a probability of fetus origin is calculated for each fragment based on current parameters of distributions. Secondly, parameters of the model are calculated from novel probabilities of fetus origin. Then first step is executed again with new parameter estimates, second step with improvement of parameters, and so on. Each iteration continuously improves probability of model parameters and observed data. The iterations stop when the change to the parameters is smaller than some prescribed constant, for example 0.000001.

## 1.4 INTERPRETING THE RESULTS

Once an EM algorithm is finished on a particular Bayesian network, we can calculate posterior probability of the observed data. We do this for all four Bayesian networks (recall that we have Bayesian network for four categories: aneuploid male, aneuploid female, euploid male, euploid female; these networks do not differ in the structure but in the setting of initial parameters like $LEN\text{-}DIST$ and $CHR\text{-}DIST$. Finally, for the examined case we choose the most probable class among these four as a result of the method.

## 1.5 FETAL FRACTION

Fetal fraction of cfDNA can be directly determined from the EM adjusted Bayesian network(s) (network parameter $f$) which we selected as the most probable case.

EXAMPLES

**Example 1**

*Sequencing techniques used in the realization of the invention*

This particular example presents preferred embodiment of the sample preparation and sequencing. The person skilled in the art is aware of the fact that most of the steps described further can be modified in respect to various factors, for example reagents used in the method, established practice of the laboratory and the final purpose of the analysis.

*Sequence analysis*

Massively parallel sequencing is necessary for the application of the method according of the invention. The method was specifically developed and validated for small benchtop next generation sequencing systems to allow low initial costs for NIPT service lab setup. The method was validated on NextSeq500 system (Illumina, Inc., San Diego, CA, USA).

Commercially available sequencing devices together with corresponding protocols and reagents recommended by the supplier, were used in the illustrative example, however, the person skilled in the art is aware of number of various sequencing methods and their variations, which could be also used in practice of the present invention.

Illumina kit NextSeq 500/550 High Output v2 kit (75 cycles) was used. Paired-end sequencing is required in this implemenatation to allow interpretation of results due to determination of fragment sizes which is part of the method. A read setting of 2 x 35bp was used. Overall, 111 samples were used with average number of reads of 5,979,689, std 3,242,203, min 1,184,843, max 17,191,421 for method evaluation.

***Full procedure of sample preparation for NextSeq500 (Illumina Inc., San Diego, CA, USA)) sequencer and analysis***

Complete method of obtaining the sequence data is described bellow with all laboratory protocols for specific steps of the method. The full process described here is substantially known to the persons skilled in the art of molecular biology, bioinformatics and prenatal testing and such a person is aware of the possible modifications of the procedure.

SAMPLING OF PERIPHERAL BLOOD

a) A 10ml sample of the peripheral venous blood of the mother taken into a special sampling tube PAXgene® Blood ccfDNA (PreAnalytiX GmbH, Feldbachstrasse, 8634 Hombrexticon, H) or other similar.
b) Instruction for blood taking:

i) Blood for the purposes of the test should be preferably taken from the 12th week of pregnancy (not before the 10th week of pregnancy ended).
ii) The sample must be taken by a female medical personnel.
iii) For the sampling, it is necessary to use the sampling tube specified above; it is prohibited to add any additional substances or to make any other interventions.
iv) During sampling, the tube must be filled within two minutes. Overfilling or insufficient filling of the tube causes incorrect results of the analysis.
v) Given the content of chemical substances in the sampling tube, additional measures must be taken during the taking of venous blood to prevent reverse suction of the tube contents into the patient's blood system.
vi) The sampling tube must be closed immediately after blood taking and the tube content must be mixed by smooth rotation eight- to ten-times. Improper and late rotation may cause incorrect or non-informative results. The tube content must not be mixed fast or vortexed. One rotation means full rotation by 180°and back.

c) Sample transportation and storage:

i) The sample must be stored and transported protected against damage at the temperature between 6°C-37°C before further processing

PLASMA SEPARATION AND DNA ISOLATION

Laboratory procedures

1. Plasma separation

i) It is required to ensure male human DNA contamination free environment with decontamination measures (PCR box with UV). Separate room for plasma isolation is recommended for effective processing of samples.
ii) It is recommended to carry out all steps be carried out by female personnel (method relies on Y chromosome counting within several algorithms and so it is sensitive to male DNA contamination)
iii) Single use tips must be used in each pipetting step.
iv) Whole blood barcode label must be scanned upon arrival at the time of processing.
v) Check the blood visually for signs of haemolysis.
vi) Centrifuge the whole blood at 2200 x g/8min
vii) Transfer plasma carefully into new 2 mL eppendorf tube (DNA LoBind)

viii) Centrifuge plasma at 20000 x g/8 min

ix) Transfer plasma carefully into 1,5 mL eppendorf tubes (DNA LoBind). Special care must be taken to prevent transfer of white blood cells from buffy coat.

x) Prepare aliquots of 500 uL volume for subsequent steps in 1,5 mL eppendorf tubes (DNA LoBind).

xi) Each aliquot must be equipped with day code label generated within NIPT app.

xii) Store at -20°C.

2. Isolation of DNA

a) We recommend using of MagMax™ Cell-Free Isolation Kit by Life Technologies for optimal yield, shorter hands on and total time and easy upgrade to automation

b) We recommend processing of plasma samples immediately

c) Plasma isolated from PAXgene tubes does not require use of proteinase K treatment within sample processing.

d) Prepare mastermix in 15 mL falcon tube with MagMax Cell Free DNA Lysis/Binding Solution (lysis byffer) and MagMax Cell Free DNA Magnetic beads calculated according to following table.

|  | 1mL plasma | 0.5ml plasma |
| --- | --- | --- |
| Lysis/binding solution | 1.25 mL | 0.625 mL |
| Magnetic beads | 15 uL | 7.5 uL |

e) Add 633 uL of prepared Lysis/Binding solution into 1.5 mL Eppendorf tube (DNA LoBind) with plasma aliquot, vortex for 10 min on Eppendorf Mix Mate shaker (2000 rpm) .

f) Move samples to magnetic stand and incubate for 5 min.

g) Discard supernatant and add 800 ul MagMax Cell Free DNA Wash solution, vortex for 30 sec on Eppendorf Mix Mate shaker (2000 rpm) .

h) Transfer samples to magnetic stand and incubate for 2 min.

i) Discard supernatant and add 800 uL of 80% ethanol. Transfer to magnetic stand and shortly incubate.

j) Repeat previous step 1x.

k) Let magnetic beads dry for 5 min and add 33 uL of ultrapure water, vortex 5 min.

l) Transfer samples to magnetic stand and incubate for 2 min.

m) Transfer final volume of 32 uL of supernatant into new eppendorf tube with daycode and proceed to quality control step (QC Qubit 2ul, priprava DNA knižnice 30ul)

n) Quality control step - concentration of isolated DNA is measured on Qubit 2.0 system using Qubit® dsDNA HS Assay Kit. Concentration over 0.3 ng/uL may be due to lysis of maternal cells and does decrease performance of the test. Such samples are not recommended for using in subsequent steps.

o) Isolated DNA can be stored at -20°C.

p) Isolated DNA in 1.5 mL Eppendorf tubes is transferred to NGS library lab. When transportation is used, always keep samples at +4°C or -20°C. It is necessary to ensures proper physical protection of sample tubes to prevent opening or cracking.

NEXT GENERATION SEQUENCING LIBRARY PREPARATION

Procedures - Library preparation (for Illumina Nexseq system)

a. Measure the concentration of isolated DNA using Qubit dsDNA HS Assay kit. "To low" concentration measurement result is OK, carry on with subsequent steps. Samples with concentration above 0.3 ng/uL must go through quality control step using Agilent 2100 and Agilent High Sensitivity DNA Kit. Do not use samples with detected DNA from lysed cells. New blood draw is needed.

b. 30 uL of sample will be used in following step

c. END REPAIR

i. Allow thawing of End repair mix2 (ERP2) at room temperature and briefly centrifuge at 600 g / 5s, incubate AmPure magnetic beads for 30 min at room temperature

ii. Use PCR plate or PCR strips for sample processing

iii. Add 30 uL of purified DNA sample to 20 uL of ERP2, vortex and briefly spin

iv. Incubate on thermal cycler at 30°C for 30 min, then cool down to 4°C

v. Centrifuge at 280 x g for 1 min

### d. SIZE SELECTION

i. Vortex magnetic beads reservoir for 1 minute. Add 1.1x of volume of magnetic beads (we recommend using Agencours AMPure beads by Beckman Coulter, the test was validated using this kit). Mix properly and spin briefly.

ii. Incubate 5 minutes at room temperature

iii. Place plate into magnetic stand for 5 min or until the liquid appears clear

iv. Transfer 105 uL of supernatant to a new 1.5 mL Eppendorf tube

v. Vortex magnetic beads reservoir for 1 minute. Add 1.8x (190ul) volume of magnetic beads to the supernatant. Mix properly and spin briefly.

vi. Incubate 5 min at room temperature.

vii. Place tubes on the magnetic stand for 5 min or until the liquid appears clear

viii. Remove and discard supernatant.

ix. First wash. Add 200 uL freshly prepared 80% EtOH and incubate 30 s.

x. Remove and discard supernatant.

xi. Second wash. Add 200 uL freshly prepared 80% EtOH and incubate 30s.

xii. Remove and discard supernatant.

xiii. Leave tubes open and allow 10 minutes for residual ethanol to evaporate.

xiv. Add 10 uL Resuspension Buffer (RSB), mix thoroughly by vortexing.

xv. Incubate 2 min at room temperature.

xvi. Place tubes on the magnetic stand for 5 min or until the liquid appears clear

xvii. Transfer 8.75 uL of the clear supernatant to the new PCR plate.

xviii. STOPPING POINT - sample can be stored for up to 1 week at -20°C.

### e. A-TAILING

i. Remove A-Tailing mix from freezer and leave 30 min at room temperature prior to use.

ii. Add 6.25 uL of A-TAILING mix, shake briefly, spin briefly.

iii. Put PCR plate to thermocycler and use the following program (preheat lid to 100°C, incubate at 37°C for 30 minutes, incubate at 70°C for 5 min, incubate at 5°C for 5 min)

iv. Centrifuge at 280 x g for 1 min

### f. ADAPTOR LIGATION

i. Remove adaptors and Stop Ligation Buffer (SL) from freezer and thaw them at room temperature, centrifuge the thawed adapters and SL at 600 x g for 5 s, remove the Ligation Mix 2 (LM 2) from freezer immediately before use, mix by inverting the tube few times and spin briefly

ii. Add 1.25 uL RSB

iii. Add 1.25 uL LM 2

iv. Add 1.25 uL of adapter (each well containing dual index in the adapter plate is diluted 1:2.5 with ultrapure water and frozen in 10 ul aliquotes, thaw-freeze cycles more than 4 times are not recommended). Vortex briefly and centrifuge at 280 x g for 1 min

v. Put PCR plate to thermocycler and use the following program (preheat lid to 100°C, incubate at 30°C for 10 minutes, incubate at 5°C for 5 min)

vi. Add 2.5 uL of Stop Ligation Buffer, mix briefly, shake and spin briefly.

vii. Transfer the total volume into new 1.5 uL Eppendorf tube.

viii. Vortex magnetic beads reservoir for 1 minute. Add 21.3 uL of magnetic beads, mix properly and spin briefly.

ix. Incubate for 5 min at room temperature.

x. Place tubes on the magnetic stand for 5 min or until the liquid appears clear

xi. Remove and discard supernatant.

xii. First wash. Add 200 uL freshly prepared 80% EtOH and incubate 30 s.

xiii. Remove and discard supernatant.

xiv. Second wash. Add 200 uL freshly prepared 80% EtOH and incubate 30 s.

xv. Remove and discard supernatant.

xvi. Leave tubes open and allow 5 minutes for residual ethanol to evaporate.

xvii. Add 26.3 uL of Resuspension Buffer (RSB), shake or mix by pipetting.

xviii. Incubate 2 min at room temperature.

xix. Place tubes on the magnetic stand. Incubate for 5 min or until the liquid appears clear

xx. Transfer 25 uL of the supernatant into a new 1.5 mL Eppendorf tube.

xxi. Vortex magnetic beads reservoir for 1 minute. Add 25 uL of magnetic beads, mix properly and shake briefly.

xxii. Incubate for 5 min at room temperature.

xxiii. Place tubes on the magnetic stand for 5 min or until the liquid appears clear

xxiv. Remove and discard supernatant.

xxv. First wash. Add 200 uL freshly prepared 80% EtOH and incubate 30 s.

xxvi. Remove and discard supernatant.

xxvii. Second wash. Add 200 uL freshly prepared 80% EtOH and incubate 30 s.

xxviii. Remove and discard supernatant.

xxix. Leave tubes open and allow 5 minutes for residual ethanol to evaporate.

xxx. Add 13.75 uL of Resuspension Buffer (RSB), shake or mix properly.

xxxi. Incubate 2 min at room temperature.

xxxii. Place tubes on the magnetic stand. Incubate for 5 min or until the liquid appears clear

xxxiii. Transfer 12.5 uL of the supernatant to a new PCR plate.

xxxiv. POSSIBLE STOPPING POINT - sample can be stored for up to 1 week at -20°C.

g. LIBRARY QUANTITATION

i. Measure the final DNA library concentration by using fluorometric Qubit dsDNA HS Assay kit (ng/ul)

ii. Determine the average size of final library on an Agilent Technologies 2100 Bioanalyzer using a High Sensitivity DNA chip (bp)

iii. Normalize the final libraries into 2-4nM final library using the formula: concentration of the final library (ng)/660 x size of the final library (bp) x $10^6$

iv. Pool the final 2-4 nM libraries into the one final.

v. Denature final library with 0,2 N NaOH, mix properly and incubate 5 min at room temperature, spin briefly

vi. Add the following volume of 200mM Tris HCl, pH 7, mix properly and spin down briefly

vii. Add the following volume of prechilled hybridization buffer HT1 to the tube of denatured libraries, vortex briefly and centrifuge at 280g for 1 min. The result is a 20pM denatured library.

viii. Dilute the denatured 20 pM library solution to 1.8 pM as follows.

- 117 ul denatured library solution
- 1183 ul prechilled HT1

The total volume is 1.3 ml at 1.8pM concentration. Mix briefly and spin down

ix. Place the library on ice until ready to proceed on NextSeq500

SEQUENCING ANALYSIS ON ILLUMINA NEXSEQ500

a. Illumina NexSeq500 massively parallel sequencing system should be used.

b. Illumina NextSeq 500/550 High Output v2 kit (75 cycles) is used for sequencing analysis run

c. Paired end sequencing is performed with 2 x 35bp read setting with dual adapter reading.

d. Only FASTQ files are required for processing in following interpretation steps.

**Example 2**

**Validation of the method with T21**

To validate prediction capabilities of the model we sequenced 4 $T_{t21x}$ (with trisomic T21 female fetus) samples, 6 $T_{t21y}$ (with trisomic T21 male fetus) samples and 100 $T_{hy}$ (with euploid male fetuses) samples, making 110 samples in total. We omitted $T_{hx}$ (euploid female fetuses) because of missing information of reference fetal fraction. Another cohort of 8 adult non-pregnant women and 4 adult men were sequenced for estimation of chromosomal distributions.

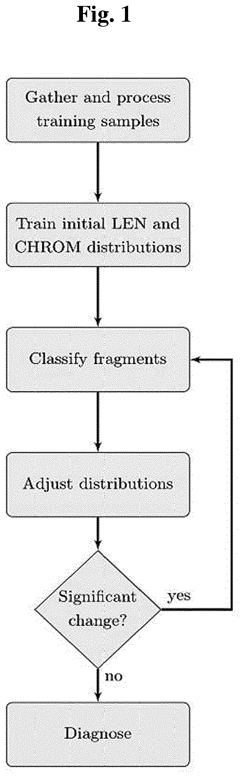

We used previously described method (Chapter 1.2 and 1.3, general scheme on Fig. 1) to initialize underlying chromosomal and fragment length distributions of the Bayesian model demonstrated on Fig. 2. Parameters were then iteratively refined using EM algorithm on 60 randomly selected samples with healthy male fetuses.

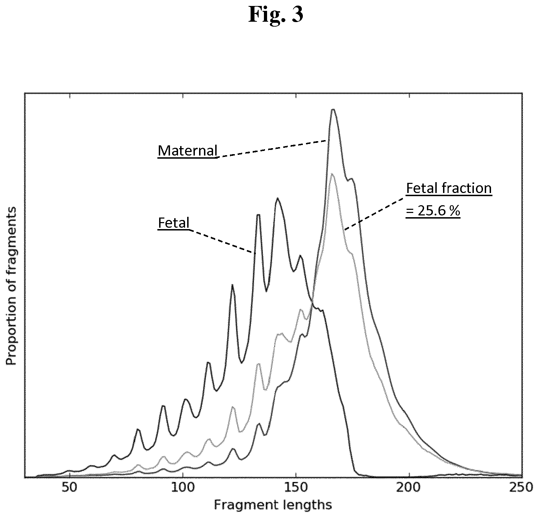

Trained distributions were used to parametrise Bayesian models for all four categories, i.e. euploid male, euploid female, trisomic male and trisomic female fetus. Fetal fraction with the maximal posterior probability of observed fragments for each model were recorded. The model with the highest probability was set as the most probable diagnosis result along with the highest ranking fetal fraction. The most probable distributions for maternal and fetal fragment lengths are shown at the Figure 3.

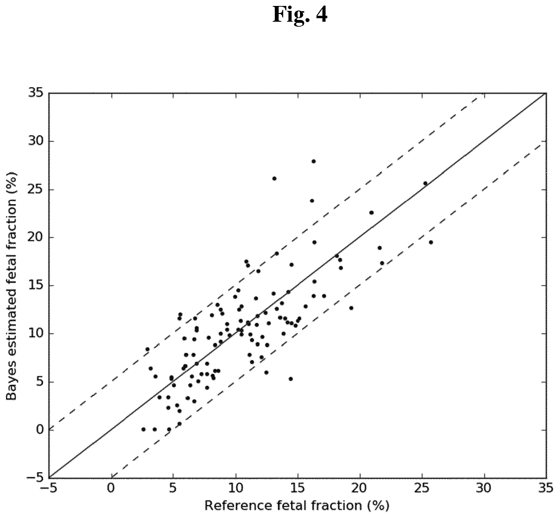

Sex and ploidy were classified correctly for all 110 samples (100% sensitivity and specificity), see Fig. 4. The most probable fetal fractions highly correlated ($r = 0.76$, $p = 3.12 \times 10^{-22}$) with reference fractions calculated from abundance of chromosome Y (male fetus) or chromosome 21 (female fetus).

To evaluate accuracy of fetal fraction prediction for euploid female fetuses, we prepared simplified Bayesian model without information of fragments chromosomal origin, using only fragment lengths. Note, that this way it is not possible to derive fetal fraction from abundance of Y or 21 chromosome fragments for male or trisomic samples, respectively. For each sample, we selected fraction with the highest posterior probability and compare it with the reference fraction. The correlation remained high even without additional information ($r = 0.77$, $p = 5.16 \times 10^{-21}$).

## Example 3

### Validation of the method with T13, T18 and X0

Prediction power of the Bayesian network was demonstrated also on additionally sequenced samples with aneuploidies; trisomy of chromosome 13 or Patau syndrome (1 new sample), trisomy of chromosome 18 or Edwards syndrome (2 new samples) and monosomy X or Turner syndrome (1 sample).

Automatic inference of chromosomal distributions with EM algorithm was not possible because of small number of target samples. Expected distributions for each condition were therefore constructed using the process presented in Section "Building chromosome distributions". Since fragment length distributions are independent from clinical condition, we used parameters inferred from samples in Example 2.

We tested each sample for conditions:

- euploid male
- euploid female
- boy with aneuploidy of chromosome 13
- girl with aneuploidy of chromosome 13
- boy with aneuploidy of chromosome 18
- girl with aneuploidy of chromosome 18
- boy with aneuploidy of chromosome 21
- girl with aneuploidy of chromosome 21
- girl with monosomy of chromosome X

Clinical condition of each sample was predicted correctly according to the most probable parameter set (100% sensitivity and specificity).

## References

Bianchi, Diana W, Platt, Lawrence D, Goldberg, James D, Abuhamad, Alfred Z, Sehnert, Amy J, & Rava, Richard P. 2012. Genome-wide fetal aneuploidy detection by maternal plasma DNA sequencing. Obstetrics & Gynecology, 119(5), 890-901.

Ehrich M., C. Deciu, T. Zwiefelhofer, J. A. Tynan, L. Cagasan, R. Tim, V. Lu, R. McCullough E. McCarthy, A. O. Nygren, et al. Noninvasive detection of fetal trisomy 21 by sequencing of dna in maternal blood: a study in a clinical setting. American journal of obstetrics and gynecology, 204(3):205-el, 2011.

Fan, H. C., Blumenfeld, Y. J., Chitkara, U., Hudgins, L., & Quake, S. R. (2008). Noninvasive diagnosis of fetal aneuploidy by shotgun sequencing DNA from maternal blood. Proceedings of the National Academy of Sciences, 105(42), 16266-16271.

Chiu, Rossa WK, Chan, KC Allen, Gao, Yuan, Lau, Virginia YM, Zheng, Wenli, Leung, Tak Y, Foo, Chris HF, Xie, Bin, Tsui, Nancy BY, Lun, Fiona MF, et al. 2008. Noninvasive prenatal diagnosis of fetal chromosomal aneuploidy

**EP 3 658 689 B1**

by massively parallel genomic sequencing of DNA in maternal plasma. Proceedings of the National Academy of Sciences, 105(51), 20458-20463.

Chiu, Rossa WK, Akolekar, Ranjit, Zheng, Yama WL, Leung, Tak Y, Sun, Hao, Chan, KC, Lun, Fiona MF, Go, Attie TJI, Lau, Elizabeth T, To, William WK, et al. 2011. Non-invasive prenatal assessment of trisomy 21 by multiplexed maternal plasma DNA sequencing: large scale validity study. Bmj, 342.

Jiang, K. C. Allen Chan, Gary J. W. Liao, Yama W. L. Zheng, Tak Y. Leung, Rossa W. K. Chiu, Yuk Ming Dennis Lo, Hao Sun; FetalQuant: deducing fractional fetal DNA concentration from massively parallel sequencing of DNA in maternal plasma. Bioinformatics 2012; 28 (22): 2883-2890. doi: 10.1093/bioinformatics/bts549

Kim, S. K., Hannum, G., Geis, J., Tynan, J., Hogg, G., Zhao, C., ... & Boom, D. (2015). Determination of fetal DNA fraction from the plasma of pregnant women using sequence read counts. Prenatal diagnosis, 35(8), 810-815.

B. Langmead and S. L. Salzberg. Fast gapped-read alignment with bowtie 2. Nature methods, 9(4):357-359, 2012.

Lau, Tze Kin, Chen, Fang, Pan, Xiaoyu, Pooh, Ritsuko K, Jiang, Fuman, Li, Yihan, Jiang, Hui, Li, Xuchao, Chen, Shengpei, & Zhang, Xiuqing. 2012. Noninvasive prenatal diagnosis of common fetal chromosomal aneuploidies by maternal plasma DNA sequencing. Journal of Maternal-Fetal and Neonatal Medicine, 25(8), 1370-1374.

Lo, YM, Corbetta, Noemi, Chamberlain, Paul F, Rai, Vik, Sargent, Ian L, Redman, Christopher WG, & Wainscoat, James S. 1997. Presence of fetal DNA in maternal plasma and serum. The Lancet, 350(9076), 485-487.

A. J. Sehnert, B. Rhees, D. Comstock, E. de Feo, G. Heilek, J. Burke, and R. P. Rava. Optimal detection of fetal chromosomal abnormalities by massively parallel dna sequencing of cell-free fetal DNA from maternal blood. Clinical chemistry, 57(7):1042-1049, 2011.

Straver, R., Oudejans, C., Sistermans, E. A., & Reinders, M. J. (2016). Calculating the fetal fraction for noninvasive prenatal testing based on genome-wide nucleosome profiles. Prenatal diagnosis, 36(7), 614-621.

C. Y. Stephanie, K. A. Chan, Y. W. Zheng, P. Jiang, G. J. Liao, H. Sun, R. Akolekar, T. Y. Leung, A. T. Go, J. M. van Vugt, et al. Size-based molecular diagnostics using plasma dna for noninvasive prenatal testing. Proceedings of the National Academy of Sciences, 111(23): 8583-8588, 2014.

Benjamini, Yuval, and Terence P. Speed. "Summarizing and correcting the GC content bias in high-throughput sequencing." Nucleic acids research (2012): gks001.

Liao, Can, et al. "Noninvasive prenatal diagnosis of common aneuploidies by semiconductor sequencing." Proceedings of the National Academy of Sciences 111.20 (2014): 7415-7420.

**Claims**

1. A method for determining aneuploidy and sex of fetus and corresponding fetal fraction from a maternal blood, plasma or serum sample comprising a mixture of DNA fragments of fetal and maternal origin, wherein the mixture of DNA fragments of fetal and maternal origin are circulating cell-free DNA molecules, said method comprising:

    a) performing a random sequencing on at least a portion of a plurality of the cell-free DNA molecules contained in the sample, thereby obtaining sequence information for a plurality of DNA fragments of fetal and maternal origin from a maternal sample, wherein the sequence information comprises sequence reads;
    b) mapping the sequence reads to the reference human genome;
    c) splitting the reference genome into 20kb subsequent and non-overlapping bins and distributing the mapped sequence reads to said bins according to their mapping location;
    d) optionally correcting number of reads for GC content bias;
    e) train initial distributions of selected features of DNA fragments on the set of training samples;
    f) construct a Bayesian network that includes the selected features of DNA fragments for four categories that combine sex and aneuploidy presence or absence in fetus, which are euploid male, aneuploid male, euploid female, aneuploid female, on the sample;

**17**

g) run iterative EM algorithm on the Bayesian network ;

h) based on the EM output

    a. classify DNA fragments from the sample as maternal or fetal;
    b. adjust distributions of selected features of DNA fragments ;

i) repeat steps g) and h) until the parameters of trained distribution does not significantly change between iterations, e.g. sum of differences is below 0.000001

j) calculate the probability of the selected features of DNA fragments in the final Bayesian network;

k) for each category find fetal fraction with highest posterior probability;

l) select the most probable category for the sample to determine the classification of the sample as one from the said categories euploid male, aneuploid male, euploid female, aneuploid female;

wherein Bayesian network for each said category which includes selected features of DNA fragment, is constructed with the set of training samples comprising set of pregnant women with euploid female fetus and set of pregnant women with euploid male fetus using the same steps as in said steps a) to i) and provides the initial distributions of the selected features of DNA fragments,

where in step e) the selected features of DNA fragment are fragment length, LEN, and chromosome of origin, CHROM, and

the set of training samples comprises

    set of pregnant women with euploid female fetus;
    set of pregnant women with euploid male fetus;
    set of adult males; and
    set of non-pregnant females.

2. The method according to claim 1 , where the distribution of chromosome of origin is determined on the basis of fractional autosomal representation, FAR, and fractional chromosomal distribution, FCR, and distribution of the fragment length is determined on the basis of fractional length distribution, FLD.

3. The method according to claim 1 or 2, where aneuploidy is trisomy of chromosome 13, trisomy of chromosome 18 or trisomy of chromosome 21.

4. A computer implemented method based on Bayesian network for determining aneuploidy and sex of fetus and corresponding fetal fraction, from random sequencing on at least a portion of a plurality of the DNA fragments contained in the sample of maternal blood, plasma or serum sample comprising a mixture of DNA fragments of fetal and maternal origin, wherein the mixture of DNA fragments of fetal and maternal origin are circulating cell-free DNA molecules, and from random sequencing on at least a portion of a plurality of the cell-free DNA molecules contained in the training sample, said method comprises the steps b) to 1) from the method of claim 1, wherein for each said category the Bayesian network including selected features of DNA fragment is constructed on the set of training samples comprising at least set of pregnant women with euploid female fetus and set of pregnant women with euploid male fetus using the same steps as in said steps a) to i) and provides the initial distributions of the selected features of DNA fragment,

where in step e) the selected features of DNA fragment are fragment length, LEN, and chromosome of origin, CHROM, and

the set of training samples comprises

    set of pregnant women with euploid female fetus;
    set of pregnant women with euploid male fetus;
    set of adult males; and
    set of non-pregnant females.

5. The computer implemented method according to claim 4, where the distribution of chromosome of origin is determined on the basis of fractional autosomal representation, FAR, and fractional chromosomal distribution, FCR, and distribution of the fragment length is determined on the basis of fractional length distribution, FLD.

6. The computer implemented method according to claim 4 or 5, where aneuploidy is trisomy of chromosome 13, trisomy of chromosome 18 or trisomy of chromosome 21

7. A computer program product comprising a computer readable medium comprising a plurality of instructions, which when the program is executed on a computer, cause the computer to perform the method according to any one of claims 4 to 6.

**Patentansprüche**

1. Verfahren zur Bestimmung von Aneuploidie und Geschlecht eines Fötus und eines entsprechenden fötalen Anteils aus einer mütterlichen Blut-, Plasma- oder Serumprobe, die eine Mischung von DNA-Fragmenten fötalen und mütterlichen Ursprungs umfasst, wobei die Mischung von DNA-Fragmenten fötalen und mütterlichen Ursprungs zirkulierende zellfreie DNA-Moleküle sind, wobei das Verfahren umfasst:

   a) Durchführen einer zufälligen Sequenzierung an mindestens einem Teil einer Vielzahl der in der Probe enthaltenen zellfreien DNA-Moleküle, wodurch Sequenzinformationen für eine Vielzahl von DNA-Fragmenten fötalen und mütterlichen Ursprungs aus einer mütterlichen Probe erhalten werden, wobei die Sequenzinformationen Sequenzabschnitte umfassen;
   b) Zuordnen der Sequenzabschnitte zu dem Referenz-Humangenom;
   c) Aufspalten des Referenz-Genoms in 20kb aufeinanderfolgende und nicht überlappende Regionen und Verteilen der zugeordneten Sequenzabschnitte auf die genannten Regionen entsprechend ihrer Zuordnungsstelle;
   d) Optional: Korrigieren der Anzahl der Abschnitte für GC-Gehalt-Bias;
   e) Trainieren initialer Verteilungen ausgewählter Merkmale von DNA-Fragmenten anhand einer Menge von Trainingsproben;
   f) Erstellen eines Bayes'schen Netzes für die Probe, das die ausgewählten Merkmale von DNA-Fragmenten für vier Kategorien umfasst, die das Geschlecht und das Vorhandensein oder Fehlen von Aneuploidie beim Fötus kombinieren, nämlich euploid männlich, aneuploid männlich, euploid weiblich, aneuploid weiblich;
   g) Ausführen eines iterativen EM-Algorithmus auf dem Bayes'schen Netz;
   h) basierend auf der EM-Ausgabe

      a. Klassifizieren von DNA-Fragmenten aus der Probe als mütterlich oder fötal;
      b. Anpassen der Verteilung ausgewählter Merkmale von DNA-Fragmenten;

   i) Wiederholen der Schritte g) und h), bis sich die Parameter der trainierten Verteilung zwischen den Iterationen nicht signifikant ändern, z.B. die Summe der Differenzen unter 0,000001 liegt;
   j) Berechnen der Wahrscheinlichkeit der ausgewählten Merkmale der DNA-Fragmente im finalen Bayes'schen Netz;
   k) Finden des fetalen Anteils mit der höchsten A-posteriori-Wahrscheinlichkeit für jede Kategorie;
   l) Auswählen der wahrscheinlichsten Kategorie für die Probe, um die Klassifikation der Probe als eine der genannten Kategorien euploid männlich, aneuploid männlich, euploid weiblich, aneuploid weiblich zu bestimmen;

   wobei das Bayes'sche Netz für jede der genannten Kategorien, welche ausgewählte Merkmale des DNA-Fragments enthalten, mit der Menge von Trainingsproben, die eine Menge schwangerer Frauen mit euploidem weiblichen Fötus und eine Menge schwangerer Frauen mit euploidem männlichen Fötus umfasst, unter Durchführung derselben Schritte wie in den genannten Schritten a) bis i), erstellt wird und es die initialen Verteilungen der ausgewählten Merkmale der DNA-Fragmente bereitstellt,
   wobei in Schritt e) die ausgewählten Merkmale des DNA-Fragments die Fragmentlänge (LEN) und das Ursprungschromosom (CHROM) sind und
   die Menge der Trainingsproben umfasst:

   Menge schwangerer Frauen mit euploidem weiblichen Fötus;
   Menge schwangerer Frauen mit euploidem männlichem Fötus;
   Menge erwachsener Männer; und
   Menge nicht-schwangerer Frauen.

2. Verfahren nach Anspruch 1, wobei die Verteilung des Ursprungschromosoms auf Grundlage der fraktionalen autosomalen Repräsentation (FAR) und der fraktionalen chromosomalen Verteilung (FCR) bestimmt wird, und die Verteilung der Fragmentlänge auf Grundlage der fraktionalen Längenverteilung (FLD) bestimmt wird.

3. Verfahren nach Anspruch 1 oder 2, wobei die Aneuploidie eine Trisomie des Chromosoms 13, eine Trisomie des Chromosoms 18 oder eine Trisomie des Chromosoms 21 ist.

4. Computerimplementiertes, auf einem Bayes'schen Netz basierendes Verfahren zur Bestimmung von Aneuploidie und Geschlecht des Fötus und des entsprechenden fötalen Anteils aus einer zufälligen Sequenzierung auf mindestens einem Teil einer Vielzahl von DNA-Fragmenten, die in der Probe der mütterlichen Blut-, Plasma- oder Serumprobe enthalten sind, die eine Mischung von DNA-Fragmenten fötalen und mütterlichen Ursprungs umfasst, wobei die Mischung von DNA-Fragmenten fötalen und mütterlichen Ursprungs zirkulierende zellfreie DNA-Moleküle und aus einer zufälligen Sequenzierung mindestens eines Teils einer Vielzahl der zellfreien DNA-Moleküle sind, die in der Trainingsprobe enthalten sind, und das Verfahren die Schritte b) bis 1) aus dem Verfahren nach Anspruch 1 umfasst, wobei das Bayes'sche Netz für jede der genannten Kategorien, welche ausgewählte Merkmale des DNA-Fragments enthalten, mit der Menge von Trainingsproben, die eine Menge schwangerer Frauen mit euploidem weiblichen Fötus und eine Menge schwangerer Frauen mit euploidem männlichen Fötus umfasst, unter Durchführung derselben Schritte wie in den genannten Schritten a) bis i), erstellt wird und es die initialen Verteilungen der ausgewählten Merkmale der DNA-Fragmente bereitstellt, wobei in Schritt e) die ausgewählten Merkmale des DNA-Fragments die Fragmentlänge (LEN) und das Ursprungschromosom (CHROM) sind und die Menge der Trainingsproben umfasst:

   Menge schwangerer Frauen mit euploidem weiblichen Fötus;
   Menge schwangerer Frauen mit euploidem männlichem Fötus;
   Menge erwachsener Männer; und
   Menge nicht-schwangerer Frauen.

5. Computerimplementierte Verfahren nach Anspruch 4, wobei die Verteilung des Ursprungschromosoms auf Grundlage der fraktionalen autosomalen Repräsentation (FAR) und der fraktionalen chromosomalen Verteilung (FCR) bestimmt wird, und die Verteilung der Fragmentlänge auf Grundlage der fraktionalen Längenverteilung (FLD) bestimmt wird.

6. Computerimplementiertes Verfahren nach Anspruch 4 oder 5, wobei Aneuploidie eine Trisomie des Chromosoms 13, eine Trisomie des Chromosoms 18 oder eine Trisomie des Chromosoms 21 ist.

7. Computerprogrammprodukt, umfassend ein computerlesbares Medium, umfassend eine Vielzahl von Befehlen, die, wenn das Programm auf einem Computer ausgeführt wird, den Computer veranlassen, das Verfahren nach einem der Ansprüche 4 bis 6 auszuführen.

**Revendications**

1. Procédé pour déterminer une aneuploïdie et le sexe d'un fœtus et la fraction fœtale correspondante à partir d'un échantillon de sang, de plasma ou de sérum maternel comprenant un mélange de fragments d'ADN d'origine fœtale et maternelle, dans lequel le mélange de fragment d'ADN d'origine fœtale et maternelle sont des molécules d'ADN acellulaire circulant, ledit procédé comprenant :

   a) la réalisation d'un séquençage aléatoire sur au moins une partie d'une pluralité des molécules d'ADN acellulaire circulant contenues dans l'échantillon, en obtenant ainsi des informations de séquence pour une pluralité de fragments d'ADN d'origine fœtale et maternelle à partir d'un échantillon maternel, où les informations de séquence comprennent des lectures de séquences ;
   b) la cartographie des lectures de séquences sur le génome humain de référence ;
   c) la division du génome de référence en bins successifs et non chevauchants de 20 kb et la distribution des lectures de séquences cartographiées par rapport auxdits bins en fonction de leur emplacement de cartographie ;
   d) facultativement, la correction du nombre de lectures pour le biais de la teneur en GC ;
   e) l'apprentissage des distributions initiales des caractéristiques sélectionnées des fragments d'ADN sur l'ensemble d'échantillons d'apprentissage ;
   f) la construction d'un réseau bayésien qui comprend les caractéristiques sélectionnées des fragments d'ADN pour quatre catégories qui combinent le sexe et la présence ou l'absence d'une aneuploïdie chez le fœtus, qui sont masculin euploïde, masculin aneuploïde, féminin euploïde, féminin aneuploïde, sur l'échantillon ;

g) l'exécution d'un algorithme EM itératif sur le réseau bayésien ;
h) en se basant sur le résultat d'EM

a. la classification des fragments d'ADN de l'échantillon comme maternels ou fœtaux ;
b. l'ajustement des distributions des caractéristiques sélectionnées des fragments d'ADN ;

i) la répétition des étapes g) et h) jusqu'à ce que les paramètres de la distribution d'apprentissage ne changent pas significativement entre les itérations, par exemple que la somme des différences soit inférieure à 0,000001
j) le calcul de la probabilité des caractéristiques sélectionnées des fragments d'ADN dans le réseau bayésien final ;
k) pour chaque catégorie, l'identification de la fraction fœtale montrant la probabilité postérieure la plus élevée ;
l) la sélection de la catégorie la plus probable pour l'échantillon afin de déterminer la classification de l'échantillon comme une desdites catégories masculin euploïde, masculin aneuploïde, féminin euploïde, féminin aneuploïde ;

dans lequel le réseau bayésien pour chaque dite catégorie qui comprend des caractéristiques sélectionnées d'un fragment d'ADN, est construit avec l'ensemble d'échantillons d'apprentissage comprenant un ensemble de femmes enceintes portant un fœtus féminin euploïde et un ensemble de femmes enceintes portant un fœtus masculin euploïde en utilisant les mêmes étapes que dans lesdites étapes a) à i), et fournit les distributions initiales des caractéristiques sélectionnées des fragments d'ADN,
dans lequel à l'étape e), les caractéristiques sélectionnées du fragment d'ADN sont la longueur du fragment, LEN, et le chromosome d'origine, CHROM, et
l'ensemble d'échantillons d'apprentissage comprend

un ensemble de femmes enceintes portant un fœtus féminin euploïde ;
un ensemble de femmes enceintes portant un fœtus masculin euploïde ;
un ensemble d'hommes adultes ; et
un ensemble de femmes non enceintes.

2. Procédé selon la revendication 1, dans lequel la distribution du chromosome d'origine est déterminée en se basant sur une représentation autosomique fractionnelle, FAR, et une distribution chromosomique fractionnelle, FCR, et la distribution de la longueur du fragment est déterminée en se basant sur une distribution de longueur fractionnelle, FLD.

3. Procédé selon la revendication 1 ou 2, dans lequel l'aneuploïdie est la trisomie du chromosome 13, la trisomie du chromosome 18 ou la trisomie du chromosome 21.

4. Procédé mis en œuvre par ordinateur basé sur un réseau bayésien pour déterminer une aneuploïdie et le sexe d'un fœtus et la fraction fœtale correspondante, à partir d'un séquençage aléatoire sur au moins une partie d'une pluralité des fragments d'ADN contenus dans l'échantillon de sang, de plasma ou de sérum maternel comprenant un mélange de fragments d'ADN d'origine fœtale et maternelle, dans lequel le mélange de fragments d'ADN d'origine fœtale et maternelle sont des molécules d'ADN acellulaire circulant, et à partir d'un séquençage aléatoire sur au moins une partie d'une pluralité des molécules d'ADN acellulaire contenues dans l'échantillon d'apprentissage, ledit procédé comprenant les étapes b) à l) du procédé de la revendication 1,
dans lequel, pour chaque dite catégorie, le réseau bayésien comprenant des caractéristiques sélectionnées du fragment d'ADN est construit sur l'ensemble d'échantillons d'apprentissage comprenant au moins un ensemble de femmes enceintes portant un fœtus féminin euploïde et un ensemble de femmes enceintes portant un fœtus masculin euploïde en utilisant les mêmes étapes que dans lesdites étapes a) à i), et fournit les distributions initiales des caractéristiques sélectionnées du fragment d'ADN,
dans lequel à l'étape e), les caractéristiques sélectionnées du fragment d'ADN sont la longueur du fragment, LEN, et le chromosome d'origine, CHROM, et
l'ensemble d'échantillons d'apprentissage comprend

un ensemble de femmes enceintes portant un fœtus féminin euploïde ;
un ensemble de femmes enceintes portant un fœtus masculin euploïde ;
un ensemble d'hommes adultes ; et
un ensemble de femmes non enceintes.

5. Procédé mis en œuvre par ordinateur selon la revendication 4, dans lequel la distribution du chromosome d'origine est déterminée en se basant sur une représentation autosomique fractionnelle, FAR, et une distribution chromosomique fractionnelle, FCR, et la distribution de la longueur du fragment est déterminée en se basant sur une distribution de longueur fractionnelle, FLD.

6. Procédé mis en œuvre par ordinateur selon la revendication 4 ou 5, dans lequel l'aneuploïdie est la trisomie du chromosome 13, la trisomie du chromosome 18 ou la trisomie du chromosome 21.

7. Produit de programme informatique comprenant un support lisible par ordinateur comprenant une pluralité d'instructions qui, lorsque le programme est exécuté sur un ordinateur, entraînent l'exécution du procédé selon l'une quelconque des revendications 4 à 6 par l'ordinateur.

# Fig. 1

# Fig. 2

| Mother | Foetus |
|--------|--------|
| $1 - f$ | $f$ |

| | Length | | | |
|---|---|---|---|---|
| Origin | 1 | 2 | ... | 1000 |
| M | $LEN - DIST_0$ | | | |
| F | $LEN - DIST_1$ | | | |

| | Chromosome | | | |
|---|---|---|---|---|
| Origin | 1 | 2 | ... | 24 |
| M | $CHR - DIST_{f,h}$ | | | |
| F | $CHR - DIST_{\{x,y\},a}$ | | | |

# Fig. 3

**Fig. 4**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 2183693 A
- EP 2366031 A
- US 8296076 B
- WO 2011051283 A, Benz, M.
- WO 2016094853 A, Yu

### Non-patent literature cited in the description

- **LO et al.** *Lancet,* 1997, vol. 350, 485-487
- **BIANCHI ; DIANA W ; PLATT ; LAWRENCE D ; GOLDBERG ; JAMES D ; ABUHAMAD ; ALFRED Z ; SEHNERT ; AMY J.** Genome-wide fetal aneuploidy detection by maternal plasma DNA sequencing. *Obstetrics & Gynecology,* 2012, vol. 119 (5), 890-901
- **EHRICH M. ; C. DECIU ; T. ZWIEFELHOFER ; J. A. TYNAN ; L. CAGASAN ; R. TIM ; V. LU ; R. MCCULLOUGH E ; MCCARTHY ; A. O. NYGREN et al.** Noninvasive detection of fetal trisomy 21 by sequencing of dna in maternal blood: a study in a clinical setting. *American journal of obstetrics and gynecology,* 2011, vol. 204 (3), 205
- **FAN, H. C. ; BLUMENFELD, Y. J. ; CHITKARA, U. ; HUDGINS, L. ; QUAKE, S. R.** Noninvasive diagnosis of fetal aneuploidy by shotgun sequencing DNA from maternal blood. *Proceedings of the National Academy of Sciences,* 2008, vol. 105 (42), 16266-16271
- **CHIU ; ROSSA WK ; CHAN ; KC ALLEN ; GAO ; YUAN ; LAU ; VIRGINIA YM ; ZHENG ; WENLI.** Noninvasive prenatal diagnosis of fetal chromosomal aneuploidy by massively parallel genomic sequencing of DNA in maternal plasma. *Proceedings of the National Academy of Sciences,* 2008, vol. 105 (51), 20458-20463
- **CHIU ; ROSSA WK ; AKOLEKAR ; RANJIT ; ZHENG ; YAMA WL ; LEUNG ; TAK Y ; SUN ; HAO.** Non-invasive prenatal assessment of trisomy 21 by multiplexed maternal plasma DNA sequencing: large scale validity study. *Bmj,* 2011, 342
- **JIANG, K. C. ; ALLEN CHAN ; GARY J. W. LIAO ; YAMA W. L. ZHENG ; TAK Y. LEUNG ; ROSSA W. K. CHIU ; YUK MING DENNIS LO ; HAO SUN.** FetalQuant: deducing fractional fetal DNA concentration from massively parallel sequencing of DNA in maternal plasma. *Bioinformatics,* 2012, vol. 28 (22), 2883-2890
- **KIM, S. K. ; HANNUM, G. ; GEIS, J. ; TYNAN, J. ; HOGG, G. ; ZHAO, C., ... ; BOOM, D.** Determination of fetal DNA fraction from the plasma of pregnant women using sequence read counts. *Prenatal diagnosis,* 2015, vol. 35 (8), 810-815
- **B. LANGMEAD ; S. L. SALZBERG.** Fast gapped-read alignment with bowtie 2. *Nature methods,* 2012, vol. 9 (4), 357-359
- **LAU ; TZE KIN ; CHEN ; FANG ; PAN ; XIAOYU ; POOH ; RITSUKO K ; JIANG ; FUMAN.** Noninvasive prenatal diagnosis of common fetal chromosomal aneuploidies by maternal plasma DNA sequencing. *Journal of Maternal-Fetal and Neonatal Medicine,* 2012, vol. 25 (8), 1370-1374
- **LO, YM ; CORBETTA ; NOEMI ; CHAMBERLAIN ; PAUL F ; RAI ; VIK ; SARGENT ; IAN L ; REDMAN.** Presence of fetal DNA in maternal plasma and serum. *The Lancet,* 1997, vol. 350 (9076), 485-487
- **A. J. SEHNERT ; B. RHEES ; D. COMSTOCK ; E. DE FEO ; G. HEILEK ; J. BURKE ; R. P. RAVA.** Optimal detection of fetal chromosomal abnormalities by massively parallel dna sequencing of cell-free fetal DNA from maternal blood. *Clinical chemistry,* 2011, vol. 57 (7), 1042-1049
- **STRAVER, R. ; OUDEJANS, C. ; SISTERMANS, E. A. ; REINDERS, M. J.** Calculating the fetal fraction for noninvasive prenatal testing based on genome-wide nucleosome profiles. *Prenatal diagnosis,* 2016, vol. 36 (7), 614-621
- **C. Y. STEPHANIE ; K. A. CHAN ; Y. W. ZHENG ; P. JIANG ; G. J. LIAO ; H. SUN ; R. AKOLEKAR ; T. Y. LEUNG ; A. T. GO ; J. M. VAN VUGT et al.** Size-based molecular diagnostics using plasma dna for noninvasive prenatal testing. *Proceedings of the National Academy of Sciences,* 2014, vol. 111 (23), 8583-8588
- **BENJAMINI, YUVAL ; TERENCE P. SPEED.** Summarizing and correcting the GC content bias in high-throughput sequencing. *Nucleic acids research,* 2012, gks001

- **LIAO, CAN et al.** Noninvasive prenatal diagnosis of common aneuploidies by semiconductor sequencing. *Proceedings of the National Academy of Sciences,* 2014, vol. 111.20, 7415-7420